(19) Europäisches Patentamt
European Patent Office
Office européen des brevets

(11) Veröffentlichungsnummer: **0 198 412** B1

(12) # EUROPÄISCHE PATENTSCHRIFT

(45) Veröffentlichungstag der Patentschrift: **22.03.89**

(21) Anmeldenummer: **86104866.8**

(22) Anmeldetag: **09.04.86**

(51) Int. Cl.[4]: **C07C 93/14,** C07C 93/26, C07C 125/065, A61K 31/135, A61K 31/27, A23K 1/16

(54) **Phenäthanolaminderivate.**

(30) Priorität: **16.04.85 CH 1607/85**
**14.02.86 CH 599/86**

(43) Veröffentlichungstag der Anmeldung: **22.10.86 Patentblatt 86/43**

(45) Bekanntmachung des Hinweises auf die Patenterteilung: **22.03.89 Patentblatt 89/12**

(84) Benannte Vertragsstaaten: **AT BE CH DE FR GB IT LI LU NL SE**

(56) Entgegenhaltungen:
**EP-A- 0 101 069**
**EP-A- 0 164 700**
**WO-A-84/00956**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel(CH)**

(72) Erfinder: **Alig, Leo, Dr., Liebrütistrasse 32, CH-4303 Kaiseraugst(CH)**
Erfinder: **Müller, Marcel, Dr., Quellenweg 10, CH-4402 Frenkendorf(CH)**

(74) Vertreter: **Lederer, Franz, Dr. et al, Van der Werth, Lederer & Riederer Patentanwälte Lucile-Grahn-Strasse 22, D-8000 München 80(DE)**

EP 0 198 412 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Phenäthanolaminderivate, Verfahren zu ihrer Herstellung und pharmazeutische Präparate auf der Basis dieser Verbindungen.

Die erfindungsgemässen Phenäthanolaminderivate sind Verbindungen der Formel

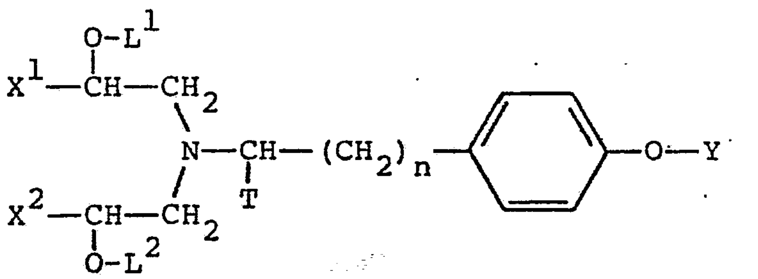

I

worin
n die Zahl 1 oder 2,
$L^1$ und $L^2$ Wasserstoff oder $C_{1-3}$-(Alkyl oder Alkoxy)carbonyl,
T Wasserstoff oder Methyl,
$X^1$ und $X^2$ Phenyl oder in m-Stellung durch Br, Cl, F, $CF_3$ oder $NO_2$ monosubstituiertes Phenyl,
Y eine Gruppe $-(CH_2)_{1-6}-O-G$, $-(CH_2)_{1-6}-CH=CH-C(O)-Z$, $-C(O)-Z$ oder $-CH(COOR'')_2$,
G $C_{1-4}$-Alkyl, $C_{1-4}$-Alkoxy-$C_{1-4}$-alkyl oder $-(CH_2)_{1-4}-Q$,
Q Phenoxy, Phenyl oder p-Fluor- oder p-Phenoxy-phenyl,
Z eine Gruppe OR oder N(R,R'), und
R und R' Wasserstoff oder $C_{1-4}$-Alkyl sind, oder R und R' zusammen mit dem N-Atom, an das sie gebunden sind, und gegebenenfalls zusammen mit einem O-Atom oder einem zusätzlichen N-Atom einen 5- oder 6-gliedrigen gesättigten Ring bilden, und
R'' $C_{1-4}$-Alkyl ist,
sowie die physiologisch verträglichen Salze davon.

Alkyl-, Alkoxy- und Alkanoylgruppen können geradkettig oder verzweigt sein; Beispiele sind Methyl, Aethyl, Propyl, Isopropyl, n-Butyl und Isobutyl; Methoxy, Aethoxy, Propoxy, Isopropoxy, Butoxy und Isobutoxy; bzw. Acetyl, Propionyl und Butyryl. Beispiele von heterocyclischen Ringen –N(R,R') sind Pyrrolidin, Piperidin, Piperazin und Morpholin.

Die Verbindungen der Formel I bilden mit Säuren Salze, die ebenfalls Gegenstand der Erfindung sind. Beispiele solcher Salze sind Salze mit physiologisch verträglichen Mineralsäuren, wie Salzsäure, Bromwasserstoffsäure, Schwefelsäure, Phosphorsäure; oder mit organischen Säuren, wie Oxalsäure, Methansulfonsäure, Essigsäure, Propionsäure, Zitronensäure, Maleinsäure, Bernsteinsäure, Aepfelsäure, Fumarsäure, Phenylessigsäure oder Salicylsäure. Carbonsäuren der Formel I können als Salze vorliegen. Beispiele für solche Salze sind Alkalimetall-, Erdalkalimetall-, Ammonium- und Aethanolammoniumsalze.

Die erfindungsgemässen Verbindungen enthalten zumindest zwei asymmetrische Kohlenstoffatome und können somit als optisch aktive Enantiomere, als Diastereomere oder als Racemate vorliegen.

Die Verbindungen der Formel I, worin G $C_{1-4}$-Alkoxyalkyl ist, sind bevorzugt. Bevorzugte Verbindungen der Formel I sind auch diejenigen, worin n die Zahl 1 ist, ferner diejenigen, in denen $L^1$ und $L^2$ Acetyl, Aethoxycarbonyl oder insbesondere Wasserstoff ist. Unter den Verbindungen, in denen T Methyl ist, sind diejenigen bevorzugt, worin das C-Atom, an dem die Methylgruppe T gebunden ist, die R-Konfiguration hat. Bevorzugte Verbindungen der Formel I sind ferner diejenigen, in denen $X^1$ und $X^2$ Phenyl, m-Fluorphenyl oder insbesondere m-Chlorphenyl sind, insbesondere diejenigen, worin das C-Atom an dem $X^1$ gebunden ist, die R-Konfiguration hat und das C-Atom, an dem $X^2$ gebunden ist, die R- oder S-Konfiguration hat. Bevorzugte Verbindungen der Formel I sind ferner diejenigen, in denen Y 2-Methoxyäthoxyäthyl, 2-p-Fluorphenäthoxyäthyl, 2-Phenoxyäthoxyäthyl, p-Phenoxybenzyloxyäthyl, Dimethylcarbamoyl, Methoxycarbonylallyl, Bis(methoxycarbonyl)-methyl, Aethoxycarbonyl oder insbesondere 2-Phenäthoxyäthyl oder 2-Aethoxyäthyl ist.

Besonders bevorzugt sind die Verbindungen der Formel I, in denen n die Zahl 1; $L^1$ und $L^2$ Wasserstoff; T Wasserstoff oder Methyl; $X^1$ und $X^2$ m-Chlorphenyl und Y 2-Phenäthoxyäthyl oder 2-Aethoxyäthyl ist, insbesondere diejenigen, worin das C-Atom, an dem $X^1$ gebunden ist, die R-Konfiguration hat und das C-Atom, an dem $X^2$ gebunden ist, die R- oder S-Konfiguration hat.

Besonders bevorzugt ist die Verbindung
α,α'-[[[p-(2-Aethoxyäthoxy)phenäthyl]imino]dimethylen]bis[(R)-m-chlorbenzylalkohol].

Weitere Beispiele von bevorzugten Verbindungen sind:
α,α'-[[[(R)-α-Methyl-p-[2-(phenäthoxy)äthoxy]phenäthyl]imino]dimethylen]bis[(R)-m-chlorbenzylalkohol],

(R)-m-Chlor-α-[[[(S)-m-chlor-β-hydroxyphenäthyl](R)-α-methyl-p-[2-(phenäthoxy)äthoxy]phenäthyl]-amino]methyl]benzylalkohol,
3-Chlor-α,α'-[[[(R)-p-(2-äthoxyäthoxy)-α-methylphenäthyl]imino]dimethylen]di-(R)-benzylalkohol,
(S)-m-Chlor-α-[[[(R)-p-(2-äthoxyäthoxy)-α-methylphenäthyl][(R)-β-hydroxyphenäthyl]amino]methyl-benzylalkohol,
(R)-m-Chlor-α-[[[(S)-m-chlor-β-hydroxyphenäthyl][p-(2-äthoxyäthoxy)phenäthyl]amino]methyl]-benzylalkohol und
(R)-m-Chlor-α-[[[(S)-m-chlor-β-hydroxyphenäthyl][p-(2-phenäthoxyäthoxy)phenäthyl]amino]methyl]-benzylalkohol.

Die erfindungsgemässen Verbindungen können dadurch hergestellt werden, dass man

a) ein dem Phenoläther der Formel I entsprechendes Phenol mit einem die Gruppe Y einführenden Mittel veräthert oder

b) ein Amin der Formel

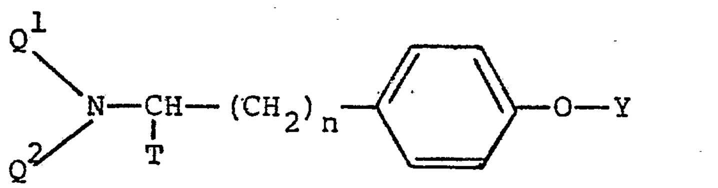

II

worin eins vor $Q^1$ und $Q^2$ Wasserstoff und das andere Wasserstoff oder eine Gruppe Q der Formel

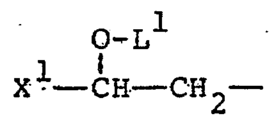 oder 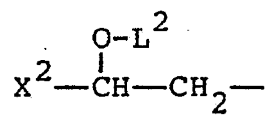

ist, mit einem die Gruppe Q einführenden Mittel alkyliert und

c) gewünschtenfalls einen in einer Gruppe Y des Reaktionsproduktes enthaltenen reaktionsfähigen Substituenten funktionell abwandelt, gewünschtenfalls eine oder beide Hydroxygruppen in β-Stellung zum Aminstickstoffatom verestert und gewünschtenfalls eine Verbindung der Formel I in ein Salz überführt.

Beispiele von in der Variante a) zu verwendenden die Gruppe Y einführenden Mitteln sind Verbindungen der Formel W–Y, worin Y die obige Bedeutung hat und W beispielsweise Halogen, insbesondere Brom oder Chlor, oder eine Sulfonatgruppe, insbesondere Methansulfonat, ist. Die Reaktion von W–Y mit einem dem herzustellenden Phenoläther der Formel I entsprechenden Ausgangsphenol kann man in an sich bekannter Weise durchführen, z.B. in einem Lösungsmittel, wie Dimethylsulfoxyd, Aceton, Tetrahydrofuran oder n-Propanol, in Gegenwart einer Base, wie Kaliumhydroxyd, Kaliumcarbonat, Kaliumt-butylat oder Triäthylamin, gegebenenfalls unter Argon bei einer Temperatur bis zur Rückflusstemperatur des Reaktionsgemisches.

Beispiele von bei der Variante b) die Gruppe Q einführenden Mittel sind Epoxyde der Formel

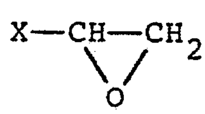

III

oder Halogenide der Formel $X–CHOH–CH_2–W$ oder $X–CO–CH_2–W$, worin X die gleiche Bedeutung wie $X^1$ oder $X^2$ hat und W Halogen, insbesondere Brom oder Chlor, ist. Die Variante b) kann man in an sich bekannter Weise bewerkstelligen, z.B. wie beschrieben in der europäischen Patentanmeldung 101069A1, zweckmässig unter Erhitzen in einem geeigneten Lösungsmittel. So lässt man das Amin II und das Epoxyd vorzugsweise in einem inerten organischen Lösungsmittel reagieren, z.B. Dimethylsulfoxid (DMSO), Acetonitril oder einem Äther, wie Tetrahydrofuran oder Dioxan, oder einem Alkohol, wie Äthanol, bei einer Temperatur zwischen 60°C und dem Siedepunkt des Reaktionsgemisches. Wenn man an Stelle des Epoxyds das Halogenid einsetzt, kann man bei einer Temperatur bis zu 200°C arbeiten in einem inerten organischen Lösungsmittel, wie einem halogenierten Kohlenwasserstoff, z.B. Chloroform. Beim Einsetzen eines Halogenids der Formel $X–CO–CH_2–W$ entsteht ein Zwischenprodukt, in dem die Ketogruppe X–CO– zur Alkoholgruppe X–CHOH– reduziert werden muss. Diese Reaktion kann man mit einem komplexen Metallhydrid, wie $NaBH_4$, in einem Lösungsmittel, wie einem Alkanol, z.B. Methanol, bei etwa 20–30°C durchführen.

Gewünschtenfalls kann man einen in einer Gruppe Y des Reaktionsproduktes der Formel I enthaltenen reaktionsfähigen Substituenten in an sich bekannter Weise funktionell abwandeln. So kann beispielsweise eine Alkoxycarbonylgruppe –C(O)–Z, z.B. eine Methoxycarbonylgruppe, mittels einer wässrigen Ammoniaklösung oder mittels Methylamin in einem Lösungsmittel, wie Aethanol, in eine Carbamoyl- bzw. Methylcarbamoylgruppe umgewandelt werden.

Gewünschtenfalls kann man eine oder beide Hydroxygruppen in β-Stellung zum Aminstickstoffatom in an sich bekannter Weise, z.B. mit einem Alkancarbonsäureanhydrid, wie Acetanhydrid, in Gegenwart einer Base, wie Pyridin, zu einer Alkanoyloxy- oder Alkoxycarbonyloxygruppe $-O-L^1$ oder $-O-L^2$, z.B. zu einer Acetoxy- oder Aethoxycarbonyloxygruppe, verestern.

Die den Phenoläthern der Formel I entsprechenden Ausgangsphenole für die Variante a) kann man in an sich bekannter Weise herstellen, z.B. wie weiter oben beschrieben im Zusammenhang mit der Variante b), nämlich durch Alkylierung eines dem Phenoläther der Formel II entsprechenden Phenols mit einem die Gruppe Q einführenden Mittel.

So wurden zur Herstellung des Ausgangsphenols im nachstehenden Beispiel 1 10,0 g p-[(R)-2-Aminopropyl]phenol und 8,8 g R-Styroloxid in 300 ml DMSO 24 Stunden auf 100°C erhitzt. Dann wurde nochmals 17,5 g R-Styroloxid zugegeben und weitere 24 Stunden auf 100°C erwärmt. Das Lösungsmittel wurde dann im Hochvakuum abgedampft und der Rückstand wurde mit Chloroform/n-Propanol/ges. NH# (1000:5:0,2) auf 1 kg Silicagel chromatographiert. Die dünnschichtchromatographisch einheitlichen Fraktionen wurden vereinigt. Man erhielt 13 g amorphes p-[(R)-2-[Bis[(R)-β-hydroxyphenäthyl]amino]propyl]-phenol, $[\alpha]_D^{20}$ = –76° (c = 1,0 in Methanol), ε@@^= 10180.

In Analogie dazu wurden folgende Ausgangsphenole erhalten:

aus Tyramin und R-Styroloxid reines, amorphes α,α'-[[(p-Hydroxyphenäthyl)imino]dimethylen]di-(R)-benzylalkohol, $[\alpha]_D^{20}$ = –53° (c = 1,0 in Methanol), das Ausgangsphenol im Beispiel 2f

aus p-[(R)-2-Aminopropyl]phenol und m-Chlor-styroloxid das p-[(R)-2-[Bis[(R,S)-β-hydroxy-(3-chlorphenäthyl)]amino]propyl]phenol, $[\alpha]_D^{20}$ = –46° (c = 0,5 in Methanol), das Ausgangsphenol im Beispiel 2g

aus p-[(R)-2-Aminopropyl]phenol und m-Trifluormethyl-styroloxid das α,α'-[[[(R)-p-Hydroxy -α-methylphenäthyl]imino]dimethylen]bis[m-(trifluormethyl)benzylalkohol], $[\alpha]_D^{20}$ = –29° (c = 1,0 in Methanol), das Ausgangsphenol im Beispiel 2h

aus p-[(R)-2-Aminopropyl]phenol und m-Nitro-styroloxid amorphes α,α'-[[[(R)-p-Hydroxy-α-methylphenäthyl]imino]dimethylen]bis[(RS)-m-nitrobenzylalkohol], das Ausgangsphenol im Beispiel 2i

aus p-[(R)-2-[[(R)-β-Hydroxyphenäthyl]amino]propyl]phenol und (S)-Styroloxid reines, amorphes (R)-α-[[[(R)-p-Hydroxy-α-methylphenäthyl][(S)-β -hydroxyphenäthyl]amino]methyl]benzylalkohol, $[\alpha]_D^{20}$ = –66° (c = 1,0 in Methanol), das Ausgangsphenol im Beispiel 7

aus Tyramin und m-Chlorstyroloxid reines, amorphes α,α'-[[(p-Hydroxyphenäthyl)imino]dimethylen]-bis[(RS)-m-chlorbenzylalkohol], das Ausgangsphenol im Beispiel 8a und 8b

aus Tyramin und m-Brom-styroloxid amorphes α,α'-[[(p-Hydroxyphenäthyl)imino]dimethylen]bis[(RS)-m-brombenzylalkohol], das Ausgangsphenol im Beispiel 8c

aus Tyramin und (R)-m-Chlorstyroloxid amorphes α,α'-[[(p-Hydroxyphenäthyl)imino]dimethylen]-bis[(R)-m-chlorbenzylalkohol, $[\alpha]_D^{20}$ = –22° (c = 0,8 in Methanol), das Ausgangsphenol im Beispiel 8d

aus p-[(R)-2-Aminopropyl]phenol und m-Brom-styroloxid amorphes α,α'-[[[(R)-p-Hydroxy-α-methylphenäthyl]imino]dimethylen]bis[(RS)-m-brombenzylalkohol], $[\alpha]_D^{20}$ = –30° (c = 1,0 in Methanol), das Ausgangsphenol des Beispiels 9a

aus p-[(R)-2-[[(R)-β-Hydroxyphenethyl]amino]propyl]phenol und m-Chlorstyroloxid reines, amorphes (RS)-m-Chlor-α-[[[(R)-p-hydroxy-α-methylphenäthyl][(R)-β-hydroxyphenäthyl]amino]methyl]benzylalkohol, das Ausgangsphenol im Beispiel 10a

aus p-[(R)-2-Aminopropyl]phenol und m-Fluor-styroloxid das p-[(R)-2-[Bis[(RS)-β-hydroxy-(3-fluorphenäthyl)]amino]propyl]phenol, amorph, $[\alpha]_D^{20}$ = –48° (c = 0,5 in Methanol), das Ausgangsphenol im Beispiel 10c

aus p-[(S)-2-Aminopropyl]phenol und m-Chlorstyroloxid amorphes p-[(S)-2-[Bis[(R,S)-β-hydroxy-(3-chlorphenäthyl)]amino]propyl]phenol, $[\alpha]_D^{20}$ = +40° (c = 1,0 in Methanol), das Ausgangsphenol im Beispiel 10d.

Die Ausgangsamine der Formel II kann man ebenfalls in an sich bekannter Weise herstellen, z.B. ausgehend von den den Phenoläthern der Formel II, worin $Q^1$ und $Q^2$ Wasserstoff sind, entsprechenden Phenolen, durch Monoalkylierung mit einem eine Gruppe Q einführenden Mittel und Verätherung des erhaltenen Phenols.

So wurde zur Herstellung des Ausgangsamins im nachstehenden Beispiel 11 Tyramin mit (R)-m-Chlorstyroloxid in DMSO unter Erhitzen zu (R)-m-Chlor-[[(p-hydroxyphenäthyl)amino]methyl]benzylalkohol umgesetzt und letzteres durch Umsetzung mit 2-Äthoxyäthylmethansulfonat zu (R)-m-Chlor-α-[[[(p-(2-äthoxyäthoxy)phenäthyl]amino]methyl]benzylalkohol veräthert, $[\alpha]_D^{20}$ = –20° (c = 0,5 in Methanol).

In Analogie dazu wurde erhalten (R)-m-Chlor-α-[[[p-[2-(phenäthoxy)äthoxy]phenäthyl]amino]methyl]benzylalkohol, $[\alpha]_D^{20}$ = −17° (c = 0,5 in Methanol), das Ausgangsamin im Beispiel 12.

Die erfindungsgemässen Phenäthanolaminderivate können als Wirkstoffe in pharmazeutischen Präparaten zur Behandlung der Fettsucht und/oder des Diabetes mellitus, insbesondere des obesen erwachsenen Diabetikers verwendet werden. Im Tierexperiment wurde auf Verabreichung von den erfindungsgemässen Phenäthanolaminderivaten ein gesteigerter Katabolismus, vor allem der Fette, beobachtet. Weiterhin wurde beobachtet, dass die erfindungsgemässen Phenäthanolaminderivate die Bildung von braunem Fettgewebe bei Ratten und obes-hyperglykämischen Mäusen stimulieren. Bekanntlich wird Defekten des braunen Fettgewebes eine wesentliche Rolle bei der Entstehung der Fettsucht zugeschrieben. An obes-hyperglykämischen Mäusen und an Streptozotocin-diabetischen Ratten haben die erfindungsgemässen Phenäthanolaminderivate einen ausgeprägten antidiabetischen Effekt, indem sie hypoglykämisch wirken und die Glykosurie vermindern. Die erfindungsgemässen Phenäthanolaminderivate zeigen nur eine geringe Wirkung auf Herztätigkeit und Kreislauf. Die Dosierung kann in Abhängigkeit von der Wirkungsstärke der einzelnen Verbindungen und den individuellen Bedürfnissen des Patienten 0,5–1000 mg, vorzugsweise 2–200 mg pro Tag für einen Erwachsenen betragen, wobei die Dosis als Einzeldosis oder in mehreren Dosen über den Tag verteilt verabreicht werden kann.

Ferner konnte mit den erfindungsgemässen Phenäthanolaminderivaten im Tierexperiment eine Erhöhung des Körper-Proteingehaltes und eine Erniedrigung des Fettgehalts nachgewiesen werden. Die erfindungsgemässen Phenäthanolaminderivate führen demnach zu einer Erhöhung der mageren Körpermasse auf Kosten von Fett. Daher können die erfindungsgemässen Phenäthanolaminderivate zunächst in der Humanmedizin zur Behandlung von Zuständen, die mit erhöhtem Proteinabbau verbunden sind, z.B. bei Rekonvaleszenz nach Operationen, verwendet werden. Dabei sind die Verabreichungsdosen die gleichen wie bei der Behandlung der Fettsucht und/oder des Diabetes mellitus.

Die erfindungsgemässen Phenäthanolaminderivate können auch in der Ernährung von Masttieren, wie Rindern, Schweinen, Schafen und Geflügel, Verwendung finden. Dabei können die Verabreichungsdosen und Verabreichungsformen die gleichen sein wie für Vitamine. Die erfindungsgemässen Phenäthanolaminderivate können auch als Futterzusatz in Dosen von 0,01–100 mg/kg je nach Substanz, Tierart und Alter eingesetzt werden.

Die pharmazeutischen Präparate enthalten den Wirkstoff zusammen mit einem verträglichen pharmazeutischen, organischen oder anorganischen Trägermaterial, wie z.B. Wasser, Gelatine, Gummi arabicum, Lactose, Stärke, Magnesiumstearat, Talk, pflanzliche Öle, Polyalkylenglykole, Vaseline und dergleichen. Die pharmazeutischen Präparate werden vorzugsweise oral, z.B. in Form von Tabletten, Kapseln, Pillen, Pulver, Granulaten, Lösungen, Sirupen, Suspensionen, Elixieren und dergleichen verabreicht. Die Verabreichung kann aber auch parenteral, z.B. in Form von sterilen Lösungen, Suspensionen oder Emulsionen, erfolgen. Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die Aktivität der neuen Verbindungen der Formel I wird aus den nachstehenden Versuchsresultaten deutlich:

1) Wirkung auf den Sauerstoffverbrauch

Männliche Albinoratten im Gewicht von 160–180 g wurden nach 24 Stunden Fasten in Stoffwechselkäfige gesetzt. Die Käfige wurden mit konstant 6 Liter Raumluft/Minute, die bei einem Taupunkt von 11°C äquilibriert wurde, belüftet. Von der Abluft wurde nach erneuter Aequilibrierung während Perioden von jeweils 14 Minuten Proben gesammelt und der Sauerstoff- und $CO_2$-Gehalt analysiert. Nach einer Anpassungszeit von 4 Stunden erhielten die in Gruppen zu 6 aufgeteilten Tiere entweder Placebo (5% Gummi arabicum) oder die Testsubstanz (suspendiert in 5% Gummi arabicum) per os. Danach wurden 12 Stunden lang die Bestimmungen durchgeführt. In Tabelle I ist der Prozentsatz des gemittelten Sauerstoffverbrauchs nach Medikation während der ersten 3 Stunden und der gesamten Versuchsdauer (12 Stunden) vom Sauerstoffverbrauch der Anpassungsperiode angegeben, wobei entsprechende Korrekturen für Änderungen in der Placebo-Gruppe berücksichtigt wurden.

Tabelle I

| Verbindung hergestellt in Beispiel Nr. | Dosis µM/kg | $O_2$-Verbrauch % vom Wert der Vorperiode 1.–3. Stunde | 1.–12. Stunde |
|---|---|---|---|
| 1 | 1 | 147 | 117 |
| 2a | 0,3 | 152 | 114 |
| 2b | 1 | 166 | 119 |
| 2c | 1 | 167 | 122 |
| 2e | 1 | 145 | 116 |
| 2f | 1 | 142 | 112 |
| 2g1 | 0,3 | 137 | 117 |
| 2g2 | 1 | 163 | 131 |
| 2g3 | 3 | 138 | 119 |
| 4 | 0,3 | 123 | 110 |
| 8d | 3 | 172 | 147 |
| 10a1 | 0,3 | 154 | 117 |
| 10a2 | 3 | 183 | 141 |
| 11 | 3 | 181 | 140 |
| 12a | 1 | 143 | 116 |

2) Wirkung auf die Urin- und Blutglukose und die Bildung von Braunem Fettgewebe

Weibliche hyperglykämische Fettmäuse wurden an eine auf 3 g/Tag/Tier begrenzte Futtermenge angepasst. Die Testverbindungen (suspendiert in 5% Gummi arabicum) oder Placebo (5% Gummi arabicum) wurden während 15 Tagen zweimal täglich oral verabreicht. Urin wurde während 6 Tagen wöchentlich gesammelt und Uringlukose bestimmt. Blutglukose und das Gewicht des interskapulären Braunen Fettgewebes wurden am Versuchsende bestimmt.

Die Versuchsresultate sind in Tabelle II als Prozentsätze der Kontrollwerte angegeben.

Tabelle II

| Verbindung hergestellt in Beispiel Nr. | Dosis µM/kg pro Tag | Uringlukose 1. Woche/2. Woche | Blutglukose | Braunes Fettgewebe |
|---|---|---|---|---|
| 2f | 90 | 4,8%/0,3% | 34% | 163% |
| | 9 | 63%/33% | 51% | 158% |
| 2g2 | 30 | 1,6%/0,6% | 33% | 144% |
| | 3 | 17,7%/2,4% | 38% | 157% |

3) Wirkung auf Urin- und Blutglukose von Streptozotocindiabetischen Ratten

In weiblichen Albinoratten (Gewicht: 130–140 g) wurde durch subkutane Injektion von Streptozotocin (70 mg/kg) ein Diabetes erzeugt. Die diabetischen Tiere wurden auf eine auf 15 g/Tag/Tier begrenzte Futtermenge adaptiert. Die Testverbindung (suspendiert in 5% Gummi arabicum) oder Placebo (5% Gummi arabicum) wurde zweimal täglich oral verabreicht. Der Urin wurde während 6 Tagen wöchentlich gesammelt und die Uringlukose bestimmt. Blutglukose wurde bei Versuchsabschluss nach 3 Wochen gemessen. Die Uringlukose-Ausscheidung der behandelten Tiere während der 3. Behandlungswoche und die Blutglukose ist in Tabelle III als Prozentsatz der Kontrollwerte angegeben.

Tabelle III

| Verbindung hergestellt in Beispiel Nr. | Dosis µM/kg pro Tag | Uringlukose 3. Woche | Blutglukose |
|---|---|---|---|
| 2g2 | 90 | 67% | 43% |

Die folgenden Beispiele erläutern die Erfindung weiter.

Beispiel 1

Zu einer Lösung von 520 mg p-[(R)-2-[Bis[(R)-β-hydroxyphenäthyl]amino]propyl]phenol in 7 ml n-Propanol gab man 186 mg Kaliumhydroxyd und 780 mg 2-(Phenäthoxy)äthylmethansulfonat und erwärmte die Mischung während 2 Stunden auf 120°. Zur Aufarbeitung wurde auf Eiswasser gegossen und mit Äthylacetat extrahiert. Der Extrakt ergab nach Waschen mit Wasser, Trocknen mit $Na_2SO_4$ und Abdampfen des Lösungsmittels 600 mg rohes Öl, das nach Chromatographie an 40 g Silicagel mit Chloroform/n-Propanol/wässrig. $NH_3$ (1000:10:1) 340 mg reines, amorphes α,α'-[[[(R)-α-Methyl-p-[2-(phenäthoxy)äthoxy]phenäthyl]imino]dimethylen]di-(R)-benzylalkohol, $[\alpha]_D^{20}$ = −66° (c = 1,0 in Methanol); $\varepsilon_{225}$ = 14080.

Beispiel 2

Analog Beispiel 1 erhielt man

2a) unter Verwendung von 2-Äthoxyäthylmethansulfonat α,α'-[[[(R)-p-(2-Äthoxyäthoxy)-α-methylphenäthyl]imino]dimethylen]di-(R)-benzylalkohol, amorph, $[\alpha]_D^{20}$ = −75° (c = 0,3 in Methanol), $\varepsilon_{223}$ = 11450,

2b) unter Verwendung von 2-(2-Methoxyäthoxy)äthylmethansulfonat α,α'-[[[(R)-p-[2-Methoxyäthoxy)äthoxy]-α-methylphenäthyl]imino]dimethylen]di-(R)-benzylalkohol, amorph, $[\alpha]_D^{20}$ = −75° (c = 0,5 in Methanol),

2c) unter Verwendung von 2-(p-Fluorphenäthoxy)äthylenmethansulfonat α,α'-[[(R)-α-Methyl-p-[2-(4-fluorphenäthoxy)äthoxy]phenäthyl]imino]dimethylen]di-(R)-benzylalkohol, $[\alpha]_D^{20}$ = −63° (c = 0,6 in Methanol),

2d) unter Verwendung von 2-(2-Phenoxyäthoxy)äthylmethansulfonat α,α'[[[(R)-α-Methyl-p-[2-phenoxyäthoxy)äthoxy]-phenäthyl]imino]dimethylen]di-(R)-benzylalkohol, $[\alpha]_D^{20}$ = −58° (c = 0,5 in Methanol),

2e) unter Verwendung von 2-(4-Phenoxybenzyloxy)äthylmethansulfonat α,α'-[[[(R)-α-Methyl-p-[2-[(p-phenoxybenzyl)oxy]äthoxy]phenäthyl]imino]dimethylen]di-(R)-benzylalkohol, amorph, $[\alpha]_D^{20}$ = −60° (c = 0,5 in Methanol),

2f) aus α,α'-[[(p-Hydroxyphenäthyl)imino]dimethylen]di-(R)-benzylalkohol und 2-Äthoxyäthylmethansulfonat α,α'-[[p-(2-Äthoxyäthoxy)phenäthyl]imino]dimethylen]di-(R)-benzylalkohol, amorph, $[\alpha]_D^{20}$ = −48° (c = 0,5 in Methanol),

2g) aus p-[(R)-2-[Bis[(R,S)-β-hydroxy-(3-chlorphenäthyl)amino]propyl]phenol und 2-(Phenäthoxy)-äthylmethansulfonat

2g1. α,α'-[[[(R)-α-Methyl-p-[2-(phenäthoxy)äthoxy]phenäthyl]imino]dimethylen]bis[(R)-m-chlorbenzylalkohol], amorph, $[\alpha]_D^{20}$ = −44° (c = 1,0 in Methanol),

2g2. (R)-m-Chlor-α-[[[(S)-m-chlor-β-hydroxyphenäthyl][(R)-α-methyl-p-[2-(phenäthoxy)äthoxy]-phenäthyl]amino]methyl]benzylalkohol], $[\alpha]_D^{20}$ = −41° (c = 0,4 in Methanol) und

2g3. α,α'-[[[(R)-α-Methyl-p-[2-(phenäthoxy)äthoxy]phenäthyl]imino]dimethylen]bis[(S)-m-chlorbenzylakohol], amorph, $[\alpha]_D^{20}$ = −17° (c = 1,0 in Methanol),

2h) aus α,α'-[[[(R)-p-Hydroxy-α-methylphenäthyl]imino]dimethylen]bis[m-(trifluormethyl)benzylalkohol] und 2-Äthoxyäthylmethansulfonat

2h1. α,α'-[[[(R)-p-(2-Äthoxyäthoxy)-α-methylphenäthyl]imino]dimethylen]bis[(R)-m-(trifluormethyl)-benzylalkohol], amorph, $[\alpha]_D^{20}$ = −52° (c = 0,2 in Methanol),

2h2. (R)-α-[[[(R)-p-(2-Äthoxyäthoxy)-α-methylphenäthyl][(S)-β-hydroxy-m-(trifluormethyl)phen-

äthyl)amino]methyl-m-(trifluormethyl)benzylalkohol, amorph, $[\alpha]_D^{20}$ = –37° (c = 0,4 in Methanol), und

2h3. α,α'-[[[(R)-p-(2-Äthoxyäthoxy) -α-methylphenäthyl]imino]dimethylen]bis[(S)-m-(trifluormethyl)-benzylalkohol], amorph, $[\alpha]_D^{20}$ = –4,5° (c = 1,2 in Methanol),

2i) aus α,α'-[[[(R)-p-Hydroxy-α-methylphenäthyl]imino]dimethylen]bis[(RS)-m-nitrobenzylalkohol und 2-Äthoxyäthanolmethansulfonat

2i1. α,α'-[[[(R)-p-(2-Äthoxyäthoxy)-α-methylphenäthyl]imino]dimethylen]bis[(R)-m-nitrobenzylalkohol, amorph, $[\alpha]_D^{20}$ = –54° (c = 1,0 in Methanol), und

2i2. (R)-α-[[[(R)-p-(2-Äthoxyäthoxy)-α-methylphenäthyl][(S)-β-hydroxy-m-nitrophenäthyl]amino]-methyl]-m-nitrobenzylalkohol, amorph, $[\alpha]_D^{20}$ = –43° (c = 0,5 in Methanol).

Beispiel 3

Eine Mischung von 755 mg α,α'-[[(p-Hydroxyphenäthyl)imino]dimethylen]di-(R)-benzylalkohol, 10 ml Tetrahydrofuran, 237 mg Dimethylcarbamoylchlorid, 5 mg 4-Dimethylaminopyridin und 202 mg Triäthylamin wurde 4 Stunden unter Argon zum Rückfluss erhitzt. Zur Aufarbeitung wurde abgekühlt, auf Eiswasser gegossen und mit Methylenchlorid extrahiert. Der Extrakt wurde mit NaCl-Lösung gewaschen, mit Na@SO$ getrocknet und eingedampft. Den Rückstand chromatographierte man auf Kieselgel. Mit Chloroform/n-Propanol/25% NH# (1000:5:0,5) wurden 510 mg reines, amorphes p-[2-[Bis[(R)-β-hydroxyphenäthyl]amino]äthyl]phenyldimethylcarbamat eluiert, $[\alpha]_D^{20}$ = –47° (c = 0,5 in Methanol).

Beispiel 4

Eine Mischung von 500 mg p-[(R)-2-[Bis[(R)-β-hydroxyphenäthyl]amino]propyl]phenol, 229 mg 4-Brom-2-butensäuremethylester, 72 mg Kaliumhydroxyd, eine Spur Kaliumjodid und 50 ml Aceton wurde 4 Stunden bei Raumtemperatur unter Argon gerührt. Zur Aufarbeitung goss man die Mischung auf Eiswasser und extrahierte mit Äthylacetat. Der organische Extrakt wurde mit Wasser neutral gewaschen, mit Na@SO$ getrocknet und im Vakuum eingedampft. Der Rückstand lieferte nach Chromatographie an Silicagel mit Chloroform/Propanol (99:1) 400 mg reines, amorphes 4-[p-([(R)-2-[Bis[(R)-β-hydroxyphenäthyl]amino]propyl]phenoxy]crotonsäuremethylester, $[\alpha]_D^{20}$ = –70° (c = 0,3 in Methanol); ε@) #= 43270.

Beispiel 5

783 mg p-[(R)-2-[Bis[(R)-β-hydroxyphenäthyl]amino]propyl]phenol wurden in 40 ml Aceton gelöst und mit 112 mg pulv. Kaliumhydroxid, 422 mg Brommalonsäuredimethylester und eine Spur Kaliumjodid 5 Stunden bei Raumtemperatur gerührt. Zur Aufarbeitung goss man auf Eiswasser und extrahierte mit Äthylacetat. Nach Eindampfen des Lösungmittels und Chromatographie des Rohproduktes auf Silicagel (Toluol/Äthylacetat 5:1) erhielt man 450 mg dünnschichtchromatographisch einheitliches, amorphes [p-[(R)-2-[Bis[(R)-β-hydroxyphenäthyl]amino]propyl]phenoxy]malonsäure-dimethylester; $[\alpha]_D^{20}$ = –58° (c = 0,5 in Methanol), ε @@$= 23600.

Beispiel 6

a) 1,17 g p-[(R)-2-[Bis[(R)-β-hydroxyphenäthyl]amino]propyl]phenol wurden in 100 ml Aceton gelöst und mit 357 mg Chlorameisensäureäthylester, 177 mg pulv. Kaliumhydroxid und einer Spur Kaliumjodid 10 Stunden bei Raumtemperatur gerührt. Aufarbeitung mit Äthylacetat, wie in Beispiel 8, lieferte 1,16 g öliges Rohprodukt, das mit Chloroform/n-Propanol/wäss. NH# (1000:10:1) auf 100 g Silicagel chromatographiert wurde. Die dünnschichtchromatographisch einheitlichen Fraktionen wurden vereinigt. Zuerst eluierte man 280 mg reines, amorphes p-[(R)-2-[[(R)-β-(Äthoxycarbonyl)oxy]phenäthyl][(R)-β-hydroxyphenäthyl]amino]propyl]phenäthylcarbonat; $[\alpha]_D^{20}$ = –80° (c = 0,5 in Methanol).

b) Dann eluierte man 380 mg reines, amorphes p-[(R)-2-[Bis[(R)-β-hydroxyphenäthyl]amino]propyl]-phenäthylcarbonat; $[\alpha]_D^{20}$ = –82° (c = 0,3 in Methanol).

Beispiel 7

Analog Beispiel 1 erhielt man
aus (R)-α-[[[(R)-p-Hydroxy-α-methylphenäthyl][(S)-β-hydroxyphenäthyl]amino]methyl]benzylalkohol
(R)-α-[[[(S)-β-Hydroxyphenäthyl][(R)-α-methyl-p-(2-phenäthoxyäthoxy)phenäthyl]amino]methyl]-benzylalkohol, $[\alpha]_D^{20}$ = –51° (c = 0,5 in Methanol).

Beispiel 8

Analog Beispiel 2f erhielt man

a) aus α,α'-[[(p-Hydroxyphenäthyl)imino]dimethylen]bis[(RS)-m-chlorbenzylalkohol] α,α'-[[[p-(2-Äthoxyäthoxy)phenäthyl]imino]dimethylen]bis[(RS)-m-chlorbenzylalkohol], IR-Banden bei 3396, 1610, 1598, 1575, 1511, 1246, 1124, 1067, 897, 826, 786, 693 $cm^{-1}$

b) aus α,α'-[[(p-Hydroxyphenäthyl)imino]dimethylen]bis[(RS)-m-chlorbenzylalkohol] und 2-(Phenäthoxy)äthylmethansulfonat α,α'-[[[p-[2-(Phenäthoxy)äthoxy]phenäthyl]imino]dimethylen]bis[(RS)-m-chlorbenzylalkohol], IR-Banden bei 3404, 1246, 1125, 1069, 826, 787, 749, 698 $cm^{-1}$

c) aus α,α'-[[(p-Hydroxyphenäthyl)imino]dimethylen]bis[(RS)-m-brombenzylalkohol] α,α'[[[p-(2-Äthoxyäthoxy)phenäthyl]imino]dimethylen]bis[(RS)-m-brombenzylalkohol], IR-Banden bei 3395, 2928, 2870, 1610, 1595, 1569, 1246, 1123, 1067, 784, 695 $cm^{-1}$ und

d) aus α,α'-[[(p-Hydroxyphenäthyl)imino]dimethylen]bis[(R)-m-chlorbenzylalkohol] α,α'-[[[p-(2-Äthoxyäthoxy)phenäthyl]imino]dimethylen]bis[(R)-m-chlorbenzylalkohol], $[\alpha]^{20}_{D}$ = −21° (c = 0,5 in Methanol).

Beispiel 9

Analog Beispiel 2g erhielt man

a) aus α,α'-[[[(R)-p-Hydroxy-α-methylphenäthyl]imino]dimethylen]bis[(RS)-m-brombenzylalkohol]

1. α,α'-[[[(R)-α-Methyl-p-[2-(phenäthoxy)äthoxy]phenäthyl]imino]dimethylen]bis[(R)-m-brombenzylalkohol], $[\alpha]^{20}_{D}$ = −42° (c = 0,3 in Methanol)

2. (R)-m-Brom-α-[[[(S)-m-brom-β-hydroxyphenäthyl][(R)-α-methyl-p-[(2-phenäthoxy)äthoxy]phenäthyl]amino]methyl]benzylalkohol, $[\alpha]^{20}_{D}$ = −34° (c = 0,5 in Methanol) und

3. α,α'-[[[(R)-α-Methyl-p-[2-(phenäthoxy)äthoxy]phenäthyl]imino]dimethylen]bis[(S)-m-brombenzylalkohol], $[\alpha]^{20}_{D}$ = −17° (c = 0,5 in Methanol)

b) aus α,α'-[[[(S)-p-Hydroxy-α-methylphenäthyl]imino]dimethylen]bis[(RS)-m-chlorbenzylalkohol]

1. α,α'-[[[(S)-α-Methyl-p-[2-(phenäthoxy)äthoxy]phenäthyl]imino]dimethylen]bis[(R)-m-chlorbenzylalkohol, $[\alpha]^{20}_{D}$ = +19° (c = 0,4 in Methanol) und

2. (R)-m-Chlor-α-[[[(S)-m-chlor-β-hydroxyphenäthyl][(S)-α-methyl-p-[(2-phenäthoxy)äthoxy]phenäthyl]amino]methyl]benzylalkohol, $[\alpha]^{20}_{D}$ = +45° (c = 0,3 in Methanol).

Beispiel 10

Analog Beispiel 2h erhielt man

a) aus (RS)-m-Chlor-α-[[[(R)-p-hydroxy-α-methylphenäthyl][(R)-β-hydroxyphenäthyl]amino]methyl]-benzylalkohol

1. 3-Chlor-α,α'-[[[(R)-p-(2-äthoxyäthoxy)-α-methylphenäthyl]imino]dimethylen]di-(R)-benzylalkohol, $[\alpha]^{20}_{D}$ = −68° (c = 0,9 in Methanol) und

2. (S)-m-Chlor-α-[[[(R)-p-(2-äthoxyäthoxy)-α-methylphenäthyl][(R)-β-hydroxyphenäthyl]amino]-methyl]benzylalkohol, $[\alpha]^{20}_{D}$ = −56° (c = 1,0 in Methanol),

b) aus α,α'-[[[(R)-p-Hydroxy-α-methylphenäthyl]imino]dimethylen]bis[(RS)-m-chlorbenzylalkohol]

1. α,α'-[[[(R)-p-(2-Äthoxyäthoxy)-α-methylphenäthyl]imino]dimethylen]bis[(R)-m-chlorbenzylalkohol], $[\alpha]^{20}_{D}$ = −59° (c = 0,5 in Methanol)

2. (R)-m-Chlor-α-[[[(S)-m-chlor-β-hydroxyphenäthyl][(R)-p-(2-äthoxyäthoxy)-α-methylphenäthyl]-amino]methyl]benzylalkohol, $[\alpha]^{20}_{D}$ = −39° (c = 0,4 in Methanol) und

3. α,α-[[[(R)-p-(2-Äthoxyäthoxy)-α-methylphenäthyl]imino]dimethylen]bis[(S)-m-chlorbenzylalkohol], $[\alpha]^{20}_{D}$ = −14° (c = 0,3 in Methanol)

c) aus p-[(R)-2-[Bis[(RS)-β-hydroxy-(3-fluorphenäthyl)]amino]propyl]phenol

1. α,α'-[[[(R)-p-(2-Äthoxyäthoxy)-α-methylphenäthyl]imino]dimethylen]bis[(R)-m-fluorbenzylalkohol], $[\alpha]^{20}_{D}$ = −73° (c = 1,0 in Methanol)

2. (R)-m-Fluor-α-[[[(S)-m-fluor-β-hydroxyphenäthyl][(R)-p-(2-äthoxyäthoxy)-α-methylphenäthyl]-amino]methyl]benzylalkohol, $[\alpha]_D^{20} = -59°$ (c = 1,0 in Methanol) und

3. α,α'[[[(R)-p-(2-Äthoxyäthoxy)-α-methylphenäthyl]imino]dimethylen]bis[(S)-m-fluorbenzylalkohol], $[\alpha]_D^{20} = -16°$ (c = 1,0 in Methanol)

d) aus p-[(S)-2-[Bis[(RS)-β-hydroxy-(3-chlorphenäthyl)]amino]propyl]phenol

1. α,α'-[[[(S)-p-(2-Äthoxyäthoxy)-α-methylphenäthyl]imino]dimethylen]bis[(S)-m-chlorbenzylalkohol], $[\alpha]_D^{20} = +57°$ (c = 0,7 in Methanol)

2. (R)-m-Chlor-α-[[[(S)-m-chlor-β-hydroxyphenäthyl][(S)-p-(2-äthoxyäthoxy)-α-methylphenäthyl]-amino]methyl]benzylalkohol, $[\alpha]_D^{20} = +52°$ (c = 0,5 in Methanol) und

3. α,α'[[[(S)-p-(2-Äthoxyäthoxy)-α-methylphenäthyl]imino]dimethylen]bis[(R)-m-chlorbenzylalkohol], $[\alpha]_D^{20} = +23°$ (c = 0,3 in Methanol).

Beispiel 11

Eine Lösung von 1,15 g (R)-m-Chlor-α-[[[p-(2-äthoxyäthoxy)phenäthyl]amino]methyl]benzylalkohol und 0,9 g (S)-m-Chlorstyroloxid in 50 ml DMSO wurde 20 Stunden auf 100° erwärmt. Das Reaktionsgemisch wurde im Hochvakuum bei 60° eingedampft und der Rückstand auf 150 g Silicagel chromatographiert. Mit Chloroform/n-Propanol/ges. $NH_3$-Lösung (1000:2:0,2) wurden 0,9 g reines amorphes (R)-m-Chlor-α-[[[(S)-m-chlor-β-hydroxyphenäthyl][p-(2-äthoxyäthoxy)phenäthyl]amino]methyl]benzylalkohol eluiert, IR-Banden bei 3396, 2928, 2871, 1598, 1575, 1511, 1246, 1124, 1066, 786, 693 $cm^{-1}$.

Beispiel 12

Analog Beispiel 11 erhielt man aus
(R)-m-Chlor-α-[[[p-[2-(phenäthoxy)äthoxy]phenäthyl]amino]methyl]benzylalkohol

a) (R)-m-Chlor-α-[[[(S)-m-chlor-β-hydroxyphenäthyl][p-[2-(phenäthoxy)äthoxy]phenäthyl]amino]-methyl]benzylalkohol, IR-Banden bei 3394, 1598, 1575, 1511, 1496, 1246, 1125, 1068, 894, 826, 786, 749, 698 $cm^{-1}$ und

b) unter Verwendung von (R)-m-Chlorstyroloxid
α,α'-[[[p-[2-(Phenäthoxy)äthoxy]phenäthyl]imino]dimethylen]bis[(R)-m-chlorbenzylalkohol], $[\alpha]_D^{20} = -17°$ (c = 0,3 in Methanol).

Beispiel 13

304 mg R-m-Chlor-α-[[[(S)-m-chlor-β-hydroxyphenäthyl][(R)-α-methyl-p-[2-(phenäthoxy)äthoxy]-phenäthyl]amino]methyl]benzylalkohol und 45 mg Oxalsäure wurden in 2 ml Methanol gelöst. Nach Zugabe von 8 ml Diäthyläther kristallisierten 225 mg reines Oxalat vom Smp. 134–135° aus, $[\alpha]_D^{20} = -19°$ (c = 1,0 in Methanol).

Beispiel 14

130 mg α,α'-[[[p-(2-Äthoxyäthoxy)phenäthyl]imino]dimethylen]bis[(R)-m-chlorbenzylalkohol] und 29 mg Maleinsäure wurden in 0,5 ml Methanol gelöst und anschliessend mit 5 ml Äther versetzt. Man liess 20 Stunden bei Raumtemperatur stehen und nutschte die ausgefallenen Kristalle ab. Man erhielt 77 mg kristallines Maleat, Smp. 116–118°, $[\alpha]_D^{20} = -44°$ (c = 0,6 in Methanol).

Beispiel 15

Es werden in üblicher Weise Tabletten folgender Zusammensetzung hergestellt:
Wirkstoff der Formel I, z.B.
α, α'-[[[p-(2-Äthoxyäthoxy)phenäthyl]imino]dimethylen]bis[(R)-m-chlorbenzylalkohol]: 250 mg
Lactose: 200 mg
Maisstärke: 300 mg
Maisstärkepaste: 50 mg
Calciumstearat: 5 mg
Dicalciumphosphat: 45 mg

**Patentansprüche für die Vertragsstaaten BE, CH, DE, FR, GB, IT, LI, LU, NL, SE**

1. Phenäthanolaminderivate der Formel

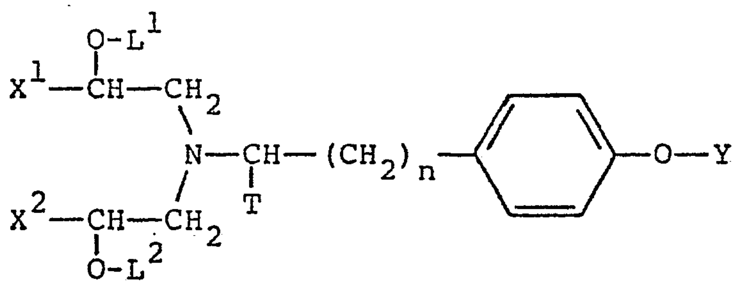

I

worin
n die Zahl 1 oder 2,
$L^1$ und $L^2$ Wasserstoff oder $C_{1-3}$-(Alkyl oder Alkoxy)carbonyl,
T Wasserstoff oder Methyl,
$X^1$ und $X^2$ Phenyl oder in m-Stellung durch Br, Cl, F, $CF_3$ oder $NO_2$ monosubstituiertes Phenyl,
Y eine Gruppe $-(CH_2)_{1-6}-O-G$, $-(CH_2)_{1-6}-CH=CH-C(O)-Z$, $-C(O)-Z$ oder $-CH(COOR'')_2$,
G $C_{1-4}$-Alkyl, $C_{1-4}$-Alkoxy-$C_{1-4}$-alkyl oder $-(CH_2)_{1-4}-Q$,
Q Phenoxy, Phenyl oder p-Fluor- oder p-Phenoxy-phenyl,
Z eine Gruppe OR oder N(R,R'), und
R und R' Wasserstoff oder $C_{1-4}$-Alkyl sind, oder R und R' zusammen mit dem N-Atom, an das sie gebunden sind, und gegebenenfalls zusammen mit einem O-Atom oder einem zusätzlichen N-Atom einen 5- oder 6-gliedrigen gesättigten Ring bilden, und
R'' $C_{1-4}$-Alkyl sind,
sowie physiologisch verträgliche Salze davon.

2. Verbindungen gemäss Anspruch 1, in denen G $C_{1-4}$-Alkoxyalkyl ist.

3. Verbindungen gemäss Anspruch 1 oder 2, in denen n die Zahl 1 ist.

4. Verbindungen gemäss Anspruch 1, 2 oder 3, in denen $L^1$ und $L^2$ Acetyl, Äthoxycarbonyl oder insbesondere Wasserstoff sind.

5. Verbindungen gemäss einem der Ansprüche 1–4, in denen T Methyl ist, insbesondere diejenigen, worin das C-Atom, an dem die Methylgruppe T gebunden ist, die R-Konfiguration hat.

6. Verbindungen gemäss einem der Ansprüche 1–5, in denen $X^1$ und $X^2$ Phenyl, m-Fluorphenyl oder insbesondere m-Chlorphenyl sind, insbesondere diejenigen, worin das C-Atom an dem $X^1$ gebunden ist, die R-Konfiguration hat und das C-Atom, an dem $X^2$ gebunden ist, die R- oder S-Konfiguration hat.

7. Verbindungen gemäss einem der Ansprüche 1–6, in denen Y 2-Methoxyäthoxyäthyl, 2-p-Fluorphenäthoxyäthyl, 2-Phenoxyäthoxyäthyl, p-Phenoxybenzyloxyäthyl, Dimethylcarbamoyl, Methoxycarbonylallyl, Bis(methoxycarbonyl)-methyl, Äthoxycarbonyl oder insbesondere 2-Phenäthoxyäthyl oder 2-Äthoxyäthyl ist.

8. Verbindungen gemäss einem der Ansprüche 1–7, in denen n die Zahl 1; $L^1$ und $L^2$ Wasserstoff; T Wasserstoff oder Methyl; $X^1$ und $X^2$ m-Chlorphenyl und Y 2-Phenäthoxyäthyl oder 2-Äthoxyäthyl ist, insbesondere diejenigen, worin das C-Atom, an dem $X^1$ gebunden ist, die R-Konfiguration hat und das C-Atom, an dem $X^2$ gebunden ist, die R- oder S-Konfiguration hat.

9. α,α'-[[[p-(2-Äthoxyäthoxy)phenäthyl]imino]dimethylen]bis[(R)-m-chlorbenzylalkohol].

10. Eine Verbindung gemäss einem der Ansprüche 1–8 aus der Gruppe der folgenden
α,α'-[[[(R)-α-Methyl-p-[2-(phenäthoxy)äthoxy]phenäthyl]imino]dimethylen]bis[(R)-m-chlorbenzylalkohol],
(R)-m-Chlor-α-[[[(S)-m-chlor-β-hydroxyphenäthyl](R)-α-methyl-p-[2-(phenäthoxy)äthoxy]phenäthyl]amino]methyl]benzylalkohol,
3-Chlor-α,α'-[[[(R)-p-(2-äthoxyäthoxy)-α-methylphenäthyl]imino]dimethylen]di-(R)-benzylalkohol,
(S)-m-Chlor-α-[[[(R)-p-(2-äthoxyäthoxy)-α-methylphenäthyl][(R)-β-hydroxyphenäthyl]amino]methyl]benzylalkohol,
(R)-m-Chlor-α-[[[(S)-m-chlor-β-hydroxyphenäthyl][p-(2-äthoxyäthoxy)phenäthyl]amino]methyl]benzylalkohol und
(R)-m-Chlor-α-[[[(S)-m-chlor-β-hydroxyphenäthyl][p-(2-phenäthoxyäthoxy)phenäthyl]amino]methyl]benzylalkohol.

11. Verbindungen der Formel I nach einem der Ansprüche 1–10 und physiologisch verträgliche Salze davon als Mittel zur Behandlung der Fettsucht, des Diabetes mellitus und von Zuständen, die mit erhöhtem Proteinabbau verbunden sind, bzw. als Futterzusatz für Masttiere.

12. Pharmazeutische Präparate, gekennzeichnet durch einen Gehalt an einer Verbindung der Formel I nach einem der Ansprüche 1–10 oder an einem physiologisch verträglichen Salz davon.

13. Verfahren zur Herstellung von Verbindungen der Formel I nach einem der Ansprüche 1–10 und von physiologisch verträglichen Salzen davon, dadurch gekennzeichnet, dass man

a) ein dem Phenoläther der Formel I entsprechendes Phenol mit einem die Gruppe Y einführenden Mittel veräthert oder

b) ein Amin der Formel

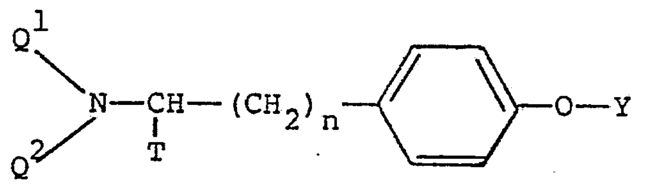

II

worin eins vor $Q^1$ und $Q^2$ Wasserstoff und das andere Wasserstoff oder eine Gruppe Q der Formel

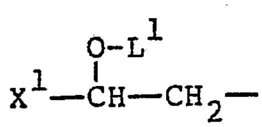

oder

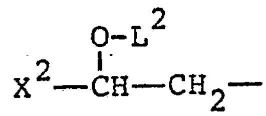

ist, mit einem die Gruppe Q einführenden Mittel alkyliert und

c) gewünschtenfalls einen in einer Gruppe Y des Reaktionsproduktes enthaltenen reaktionsfähigen Substituenten funktionell abwandelt, gewünschtenfalls eine oder beide Hydroxygruppen in β-Stellung zum Aminstickstoffatom verestert und gewünschtenfalls eine Verbindung der Formel I in ein Salz überführt.

14. Verwendung einer Verbindung der Formel I nach einem der Ansprüche 1–10 oder eines physiologisch verträglichen Salzes davon bei der Herstellung eines pharmazeutischen Präparates zur Behandlung der Fettsucht, des Diabetes mellitus und von Zuständen, die mit erhöhtem Proteinabbau verbunden sind, bzw. eines Futterzusatzes für Masttiere.

**Patentansprüche für den Vertragsstaat : AT**

1. Verfahren zur Herstellung von Phenäthanolaminderivaten der Formel

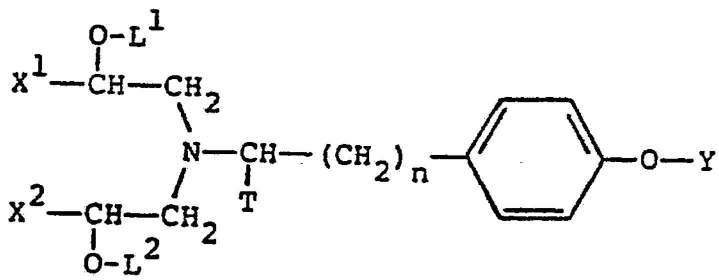

I

worin
n die Zahl 1 oder 2,
$L^1$ und $L^2$ Wasserstoff oder $C_{1-3}$-(Alkyl oder Alkoxy)carbonyl,
T Wasserstoff oder Methyl,
$X^1$ und $X^2$ Phenyl oder in m-Stellung durch Br, Cl, F, $CF_3$ oder $NO_2$ monosubstituiertes Phenyl,
Y eine Gruppe $-(CH_2)_{1-6}-O-G$, $-(CH_2)_{1-6}-CH=CH-C(O)-Z$, $-C(O)-Z$ oder $-CH(COOR'')_2$,
G $C_{1-4}$-Alkyl, $C_{1-4}$-Alkoxy-$C_{1-4}$-alkyl oder $-(CH_2)_{1-4}-Q$,
Q Phenoxy, Phenyl oder p-Fluor- oder p-Phenoxy-phenyl,
Z eine Gruppe OR oder N(R,R'), und
R und R' Wasserstoff oder $C_{1-4}$-Alkyl sind, oder R und R' zusammen mit dem N-Atom, an das sie gebunden sind, und gegebenenfalls zusammen mit einem O-Atom oder einem zusätzlichen N-Atom einen 5- oder 6-gliedrigen gesättigten Ring bilden, und
R'' $C_{1-4}$-Alkyl sind,
sowie von physiologisch verträglichen Salzen davon, dadurch gekennzeichnet, dass man

a) ein dem Phenoläther der Formel I entsprechendes Phenol mit einem die Gruppe Y einführenden Mittel veräthert oder

b) ein Amin der Formel

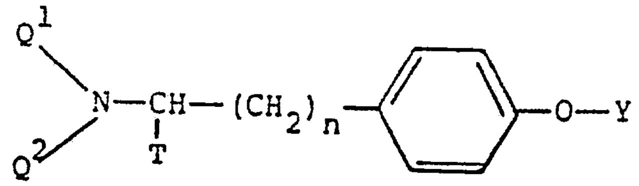

II

worin eins vor $Q_1$und $Q_2$ Wasserstoff und das andere Wasserstoff oder eine Gruppe Q der Formel

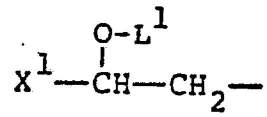

or

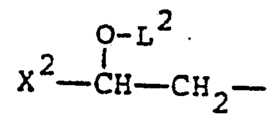

ist, mit einem die Gruppe Q einführenden Mittel alkyliert und

c) gewünschtenfalls einen in einer Gruppe Y des Reaktionsproduktes enthaltenen reaktionsfähigen Substituenten funktionell abwandelt, gewünschtenfalls eine oder beide Hydroxygruppen in β-Stellung zum Aminstickstoffatom verestert und gewünschtenfalls eine Verbindung der Formel I in ein Salz überführt.

2. Verfahren nach Anspruch1a und 1c), worin G $C_{1-6}$-Alkoxyalkyl ist.

3. Verfahren gemäss Anspruch 1 oder 2, worin n die Zahl 1 ist.

4. Verfahren gemäss Anspruch 1, 2 oder 3, worin $L_1$und $L_2$ Acetyl, Äthoxycarbonyl oder insbesondere Wasserstoff sind.

5. Verfahren gemäss einem der Ansprüche 1–4, worin T Methyl ist, insbesondere, worin das C-Atom, an dem die Methylgruppe T gebunden ist, die R-Konfiguration hat.

6. Verfahren gemäss einem der Ansprüche 1–5, worin $X_1$und $X_2$ Phenyl, m-Fluorphenyl oder insbesondere m-Chlorphenyl sind, insbesondere, worin das C-Atom an dem $X_1$gebunden ist, die R-Konfiguration hat und das C-Atom, an dem $X_2$ gebunden ist, die R- oder S-Konfiguration hat.

7. Verfahren gemäss einem der Ansprüche 1–6, worin Y 2-Methoxyäthoxyäthyl, 2-p-Fluorphenäthoxyäthyl, 2-Phenoxyäthoxyäthyl, p-Phenoxybenzyloxyäthyl, Dimethylcarbamoyl, Methoxycarbonylallyl, Bis(methoxycarbonyl)-methyl, Äthoxycarbonyl oder insbesondere 2-Phenäthoxyäthyl oder 2-Äthoxyäthyl ist.

8. Verfahren gemäss einem der Ansprüche 1–7, worin n die Zahl 1; $L_1$und $L_2$ Wasserstoff; T Wasserstoff oder Methyl; $X_1$und $X_2$ m-Chlorphenyl und Y 2-Phenäthoxyäthyl oder 2-Äthoxyäthyl ist, insbesondere, worin das C-Atom, an dem $X_1$gebunden ist, die R-Konfiguration hat und das C-Atom, an dem $X_2$ gebunden ist, die R- oder S-Konfiguration hat.

9. Verfahren nach einem der Ansprüche 1–8, dadurch gekennzeichnet, dass man α,α'-[[[p-(2-Äthoxyäthoxy)phenäthyl]imino]dimethylen]bis[(R)-m-chlorbenzylalkohol] herstellt.

10. Verfahren gemäss einem der Ansprüche 1–8, dadurch gekennzeichnet, dass man eine Verbindung aus der Gruppe der folgenden herstellt

α,α'-[[[(R)-α-Methyl-p-[2-(phenäthoxy)äthoxy]phenäthyl]imino]dimethylen]bis[(R)-m-chlorbenzylalkohol],

(R)-m-Chlor-α-[[[(S)-m-chlor-β-hydroxyphenäthyl](R)-α-methyl-p-[2-(phenäthoxy)äthoxy]phenäthyl]-amino]methyl]benzylalkohol,

3-Chlor-α,α'-[[[(R)-p-(2-äthoxyäthoxy)-α-methylphenäthyl]imino]dimethylen]di-(R)-benzylalkohol,

(S)-m-Chlor-α-[[[(R)-p-(2-äthoxyäthoxy)-α-methylphenäthyl][(R)-β-hydroxyphenäthyl]amino]methyl]-benzylalkohol,

(R)-m-Chlor-α-[[[(S)-m-chlor-β-hydroxyphenäthyl][p-(2-äthoxyäthoxy)phenäthyl]amino]methyl]benzylalkohol und

(R)-m-Chlor-α-[[[(S)-m-chlor-β-hydroxyphenäthyl][p-(2-phenäthoxyäthoxy)phenäthyl]amino]methyl]-benzylalkohol.

**Claims for the Contracting States BE, CH, DE, FR, GB, IT, LI, LU, NL, SE**

1. Phenethanolamine derivatives of the formula

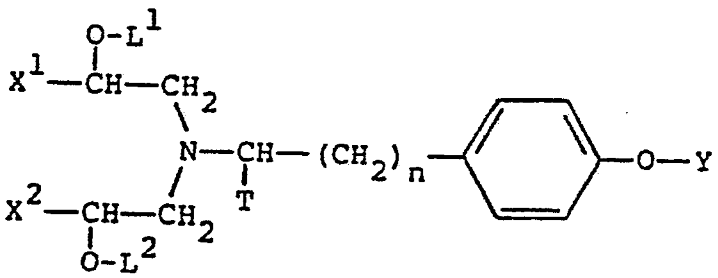

I

wherein
n is the number 1 or 2,
$L^1$ and $L^2$ are hydrogen or C!_#(alkyl or alkoxy)carbonyl,
T is hydrogen or methyl,
$X^1$ and $X^2$ are phenyl or phenyl which is monosubstituted in the m-position by Br, Cl, F, CF# or NO@,
Y is a group –(CH@)!_^O–G, –(CH@)!_^CH=CH–C(O)–Z, –C(O)–Z or –CH(COOR")@,
G is C!_$alkyl, C!_$alkoxy–C!_$alkyl or –(CH@)!_$Q,
Q is phenoxy, phenyl or p-fluoro- or p-phenoxy-phenyl,
Z is a group OR or N(R,R'), and
R and R' are hydrogen or C!_$alkyl or R and R' together with the N-atom to which they are attached from a 5- or 6-membered saturated ring which optionally contains an O-atom or an additional N-atom, and
R" is C!_$alkyl,
as well as physiologically compatible salts thereof.

2. Compounds in accordance with claim 1, in which G is C!_$alkoxyalkyl.

3. Compounds in accordance with claim 1 or 2, in which n is the number 1.

4. Compounds in accordance with claim 1, 2 or 3, in which $L^1$ and $L^2$ are acetyl, ethoxycarbonyl or especially hydrogen.

5. Compounds in accordance with any one of claims 1–4, in which T is methyl, especially those in which the C-atom to which the methyl group T is attached has the R-configuration.

6. Compounds in accordance with any one of claims 1–5, in which $X^1$ and $X^2$ are phenyl, m-fluorophenyl or especially m-chlorophenyl, particularly those in which the C-atom to which $X^1$ is attached has the R-configuration and the C-atom to which $X^2$ is attached has the R- or S-configuration.

7. Compounds in accordance with any one of claims 1–6, in which Y is 2-methoxyethoxyethyl, 2-p-fluorophenethoxyethyl, 2-phenoxyethoxyethyl, p-phenoxybenzyloxyethyl, dimethylcarbamoyl, methoxycarbonylallyl, bis(methoxycarbonyl)-methyl, ethoxycarbonyl or especially 2-phenethoxyethyl or 2-ethoxyethyl.

8. Compounds in accordance with any one of claims 1–7, in which n is the number 1; $L^1$ and $L^2$ are hydrogen; T is hydrogen or methyl; $X^1$ and $X^2$ are m-chlorophenyl and Y is 2-phenethoxyethyl or 2-ethoxyethyl, especially those in which the C-atom to which $X^1$ is attached has the R-configuration and the C-atom to which $X^2$ is attached has the R- or S-configuration.

9. α.α'–[[[p-(2-Ethoxyethoxy)phenethyl]imino]dimethylene]bis[(R)-m-chlorobenzyl alcohol].

10. A compound in accordance with any one of claims 1–8 from the following group:
α,α'-[[[(R)-α-Methyl-p-[2-(phenethoxy)ethoxy]phenethyl]imino]dimethylene]bis[(R)-m-chlorobenzyl alcohol],
(R)-m-chloro-α-[[[(S)-m-chloro-β-hydroxyphenethyl]-(R)-α-methyl-p-[2-(phenethoxy)ethoxy]phenethyl]amino]methyl]benzyl alcohol,
3-chloro-α,α'-[[[(R)-p-(2-ethoxyethoxy)-α-methylphenthyl]imino]dimethylene]di-(R)-benzyl alcohol,
(S)-m-chloro-α-[[[(R)-p-(2-ethoxyethoxy)-α-methylphenethyl][(R)-β-hydroxyphenethyl]amino]methyl]-benzyl alcohol,
(R)-m-chloro-α-[[[(S)-m-chloro-β-hydroxyphenethyl][p-(2-ethoxyethoxy)phenethyl]amino]methyl]benzyl alcohol and
(R)-m-chloro-α-[[[(S)-m-chloro-β-hydroxyphenethyl][p-(2-phenethoxyethoxy)phenethyl]amino]methyl]-benzyl alcohol.

11. Compounds of formula I according to any one of claims 1–10 and physiologically compatible salts thereof as agents for the treatment of obesity, of diabetes mellitus and of conditions which are associated with an increased protein breakdown, or as feed additives for fattening animals.

12. Pharmaceutical preparations characterized by a content of a compound of formula I according to any one of claims 1–10 or of a physiologically compatible salt thereof.

13. A process for the manufacture of compounds of formula I according to any one of claims 1–10 and of physiologically compatible salts thereof, characterized by

a) etherifying a phenol corresponding to the phenol ether of formula I with an agent which introduces the group Y, or

b) alkylating an amine of the formula

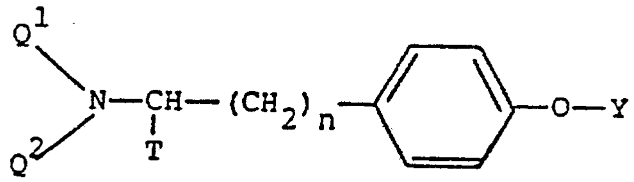

II

wherein one of $Q^1$ and $Q^2$ is hydrogen and the other is hydrogen or a group Q of the formula

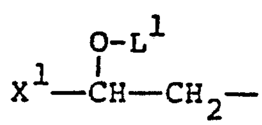

or

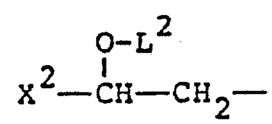

with an agent which introduces the group Q, and

c) if desired functionally modifying a reactive substituent present in a group Y of the reaction product, if desired esterifying one or both hydroxy groups in the β-position to the amine nitrogen atom and, if desired, converting a compound of formula I into a salt.

14. The use of a compound of formula I according to any one of claims 1–10 or of a physiologically compatible salt thereof in the manufacture of a pharmaceutical preparation for the treatment of obesity, of diabetes mellitus and of conditions which are associated with an increased protein breakdown, or of a feed additive for fattening animals.

**Claims for the Contracting State AT**

1. A process for the manufacture of phenethanolamine derivatives of the formula

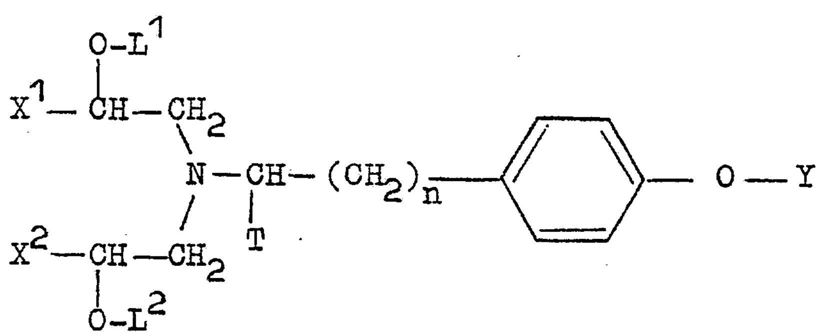

I

wherein
n is the number 1 or 2,
$L^1$ and $L^2$ are hydrogen or C!_#(alkyl or alkoxy)carbonyl,
T is hydrogen or methyl,
$X^1$ and $X^2$ are phenyl or phenyl which is monosubstituted in the m-position by Br, Cl, F, CF# or NO@,
Y is a group –(CH2)!_≏O–G, –(CH@)!_≏CH=CH–C(O)–Z, –C(O)–Z or –CH(COOR")@,
G is C!_$alkyl, C!_$alkoxy-C!_$alkyl or –(CH@)!_$Q,
Q is phenoxy, phenyl or p-fluoro- or p-phenoxy-phenyl,
Z is a group OR or N(R,R'), and
R and R' are hydrogen or C!_$alkyl or R and R' together with the N-atom to which they are attached form a 5- or 6-membered saturated ring which optionally contains an O-atom or an additional N-atom, and
R" is C!_$alkyl,
as well as of physiologically compatible salts thereof, characterized by

a) etherifying a phenol corresponding to the phenol ether of formula I with an agent which introduces the group Y, or

b) alkylating an amine of the formula

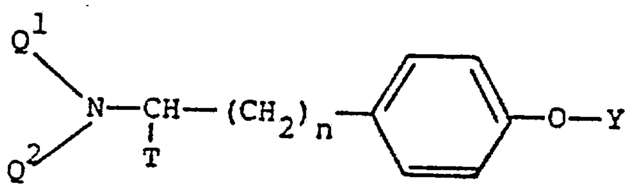

II

wherein one of $Q^1$ and $Q^2$ is hydrogen and the other is hydrogen or a group Q of the formula

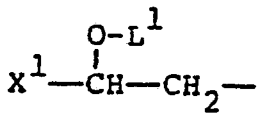

or

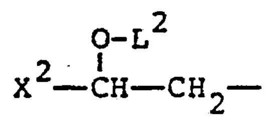

with an agent which introduces the group Q, and

c) if desired functionally modifying a reactive substituent present in a group Y of the reaction product, if desired esterifying one or both hydroxy groups in the β-position to the amine nitrogen atom and, if desired, converting a compound of formula I into a salt.

2. A process according to claim 1a) and 1c), wherein G is C!_¢alkoxyalkyl.

3. A process in accordance with claim 1 or 2, wherein n is the number 1.

4. A process in accordance with claim 1, 2 or 3, wherein $L^1$ and $L^2$ are acetyl, ethoxycarbonyl or especially hydrogen.

5. A process in accordance with any one of claims 1–4, wherein T is methyl, especially in which the C-atom to which the methyl group T is attached has the R-configuration.

6. A process in accordance with any one of claims 1–5, wherein $X^1$ and $X^2$ are phenyl, m-fluorophenyl or especially m-chlorophenyl, particularly in which the C-atom to which $X^1$ is attached has the R-configuration and the C-atom to which $X^2$ is attached has the R- or S-configuration.

7. A process in accordance with any one of claims 1–6, wherein Y is 2-methoxyethoxyethyl, 2-p-fluorophenylethoxyethyl, 2-phenoxyethoxyethyl, p-phenoxybenzyloxyethyl, dimethylcarbamoyl, methoxycarbonylallyl, bis(methoxycarbonyl)-methyl, ethoxycarbonyl or especially 2-phenoxyethyl or 2-ethoxyethyl.

8. A process in accordance with any one of claims 1–7, wherein n is the number 1; $L^1$ and $L^2$ are hydrogen; T is hydrogen or methyl; $X^1$ and $X^2$ are m-chlorophenyl and Y is 2-phenethoxyethyl or 2-ethoxyethyl, especially in which the C-atom to which $X^1$ is attached has the R-configuration and the C-atom to which $X^2$ is attached has the R- or S-configuration.

9. A process according to any one of claims 1–8, characterized in that α,α'-[[[p-(2-ethoxyethoxy)phenethyl]imino]dimethylene]bis[(R)-m-chlorobenzyl alcohol] is manufactured.

10. A process in accordance with any one of claims 1–8, characterized in that a compound from the following group α,α'-[[[(R)-α-methyl-p-[2-(phenethoxy)ethoxy]phenethyl]imino]dimethylene]bis[(R)-m-chlorobenzyl alcohol],
(R)-m-chloro-α-[[[(S)-m-chloro-β-hydroxyphenethyl]-(R)-α-methyl-p-[2-(phenethoxy)ethoxy]phenethyl]amino]methyl]benzyl alcohol,
3-chloro-α,α'-[[[(R)-p-(2-ethoxyethoxy)-α-methylphenthyl]imino]dimethylene]di-(R)-benzyl alcohol,
(S)-m-chloro-α-[[[(R)-p-(2-ethoxyethoxy)-α-methylphenethyl][(R)-β-hydroxyphenethyl]amino]methyl]-benzyl alcohol,
(R)-m-chloro-α-[[[(S)-m-chloro-β-hydroxyphenethyl][p-(2-ethoxyethoxy)phenethyl]amino]methyl]benzyl alcohol and
(R)-m-chloro-α-[[[(S)-m-chloro-β-hydroxyphenethyl][p-(2-phenethoxyethoxy)phenethyl]amino]methyl]-benzyl alcohol
is manufactured.

**Revendications pour les Etats contractants BE, CH, DE, FR, GB, IT, LI, LU, NL, SE**

1. Dérivés de la phénéthanolamine répondant à la formule

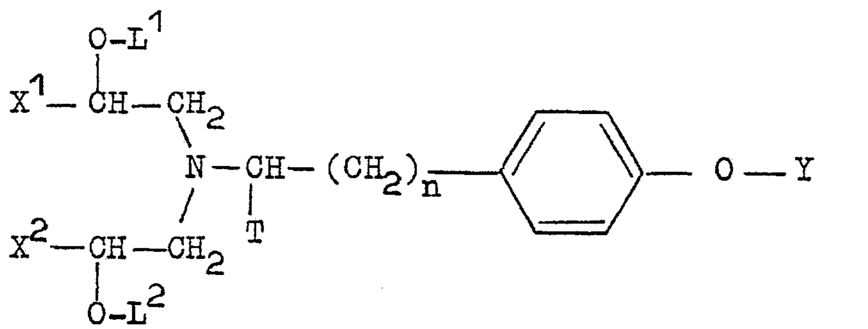

I

dans laquelle
n est égal à 1 ou 2,
$L^1$ et $L^2$ représentent l'hydrogène ou des groupes (alkyle ou alcoxy en C !-C#)-carbonyle,
T représente l'hydrogène ou un groupe méthyle,
$X^1$ et $X^2$ représentent des groupes phényle ou phényle monosubstitués en position m par Br, Cl, F, $CF_3$ ou $NO_2$,
Y représente un groupe –($CH_2$) !_^O–G, –(CH@) !_^CH=CH–C(O)–Z, –C(O)–Z ou –CH(COOR")@,
G représente un groupe alkyle en C !-C$, (alcoxy en C !-C$)-alkyle en C !-C$ ou –(CH@) !_$Q,
Q représente un groupe phénoxy, phényle ou p-fluoro- ou p-phénoxy-phényle,
Z représente un groupe OR ou N(R,R') et
R et R' représentent l'hydrogène ou des groupes alkyle en C !-C$ ou bien
R et R' forment ensemble et avec l'atome d'azote auquel ils sont reliés, et le cas échéant avec un atome d'oxygène ou un atome d'azote supplémentaire un cycle saturé à 5 ou 6 chaînons, et
R" représente un groupe alkyle en C !-C$,
et les sels de ces composés acceptables pour l'usage pharmaceutique.

2. Composés selon la revendication 1, dans lesquels G représente un groupe alcoxyalkyle en C !-C$.

3. Composés selon la revendication 1 ou 2, dans lesquels n est égal à 1.

4. Composés selon les revendications 1, 2 ou 3 dans lesquels $L^1$ et $L^2$ représentent des groupes acétyle, éthoxycarbonyle ou plus spécialement l'hydrogène.

5. Composés selon l'une des revendications 1 à 4, dans lesquels T représente un groupe méthyle, en particulier ceux dans lesquels l'atome de carbone sur lequel le groupe méthyle T est fixé, est en configuration R.

6. Composés selon l'une des revendications 1 à 5, dans lesquels $X^1$ et $X^2$ représentent des groupes phényle, m-fluorophényle ou plus spécialement m-chlorophényle, en particulier ceux dans lesquels l'atome de carbone sur lequel $X^1$ est fixé est en configuration R et l'atome de carbone sur lequel X2 est fixé est en configuration R ou S.

7. Composés selon l'une des revendications 1 à 6, dans lesquels Y représente un groupe 2-méthoxyéthoxyéthyle, 2-p-fluorophénéthoxyéthyle, 2-phénoxyéthoxyéthyle, p-phénoxybenzyloxyéthyle, diméthylcarbamoyle, méthoxycarbonylallyle, bis-(méthoxycarbonyl)-méthyle, éthoxycarbonyle ou plus spécialement 2-phénéthoxyéthyle ou 2-éthoxyéthyle.

8. Composés selon l'une des revendications 1 à 7, dans lesquels n est égal à 1; $L^1$ et $L^2$ représentent l'hydrogène; T représente l'hydrogène ou un groupe méthyle; $X^1$ et $X^2$ représentent des groupes m-chlorophényle et Y représente un groupe 2-phénéthoxyéthyle ou 2-éthoxyéthyle, en particulier ceux dans lesquels l'atome de carbone sur lequel $X^1$ est fixé est en configuration R et l'atome de carbone sur lequel $X^2$ est fixé est en configuration R ou S.

9. L'alcool α,α'-[[[p-(2-éthoxyéthoxy)phénéthyl]-imino]-diméthylène]-bis-[(R)m-chlorobenzylique].

10. Un composé selon l'une des revendications 1 à 8, du groupe des suivants:
l'alcool α,α'-[[[(R)-α-méthyl-p-[2-(phénéthoxy)-éthoxy]-phénéthyl]-imino]-diméthylène]-bis-[(R)-m-chlorobenzylique],
l'alcool (R)-m-chloro-α-[[[(S)-m-chloro-β-hydroxyphénéthyl]-(R)-α-méthyl-p-[2-(phénéthoxy)-éthoxy]-phénéthyl]-amino]-méthyl]-benzylique,
l'alcool 3-chloro-α,α'-[[[(R)-p-(2-éthoxyéthoxy)-α-méthyl-phénéthyl]-imino]-diméthylène]-di-(R)-benzylique,
l'alcool (S)-m-chloro-α-[[[(R)-p-(2-éthoxyéthoxy)-α-méthyl-phénéthyl]-[(R)-β-hydroxyphénéthyl]-amino]-méthyl]-benzylique,
l'alcool (R)-m-chloro-α-[[[(S)-m-chloro-β-hydroxyphénéthyl]-[p-(2-éthoxyéthoxy)-phénéthyl]-amino]-méthyl]-benzylique et

l'alcool (R)-m-chloro-α-[[[(S)-m-chloro-β-hydroxyphénéthyl]-[p-(2-phénéthoxyéthoxy)-phénéthyl]-amino]-méthyl]-benzylique.

11. Composés de formule I selon l'une des revendications 1 à 10 et leurs sels acceptables pour l'usage pharmaceutique en tant qu'agents pour le traitement de l'obésité, du diabète sucré et des états associés à une dégradation accrue des protéines ou en tant qu'additifs alimentaires pour des animaux à l'engrais.

12. Compositions pharmaceutiques caractérisées en ce qu'elles contiennent un composé de formule I selon l'une des revendications 1 à 10 ou un sel d'un tel composé acceptable pour l'usage pharmaceutique.

13. Procédé de préparation des composés de formule I selon l'une des revendications 1 à 10 et de leurs sels acceptables pour l'usage pharmaceutique, caractérisé en ce que

a) on éthérifie un phénol correspondant à l'éther de phénol de formule I au moyen d'un agent introduisant le groupe Y ou bien

b) on alkyle une amine de formule

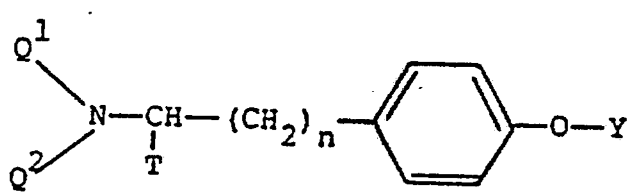

II

dans laquelle l'un des symboles $Q^1$ et $Q^2$ représente l'hydrogène et l'autre l'hydrogène ou un groupe Q de formule

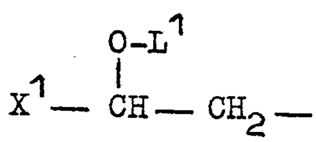

ou

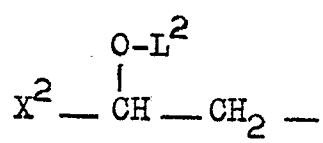

à l'aide d'un agent introduisant le groupe Q et

c) si on le désire, on soumet un substituant réactif contenu dans un groupe Y du produit de réaction à une modification fonctionnelle, si on le désire on estérifie un groupe hydroxy ou les deux groupes hydroxy en position bêta de l'atome d'azote aminé et si on le désire, on convertit un composé de formule I en un sel.

14. Utilisation d'un composé de formule I selon l'une des revendications 1 à 10 ou d'un sel d'un tel composé acceptable pour l'usage pharmaceutique pour la préparation d'une composition pharmaceutique pour le traitement de l'obésité, du diabète sucré et des états associés à une dégradation accrue des protéines, ou d'un additif aux aliments pour animaux à l'engrais.

**Revendications pour l'Etat contractant AT**

1. Procédé de préparation des dérivés de la phénéthanolamine répondant à la formule

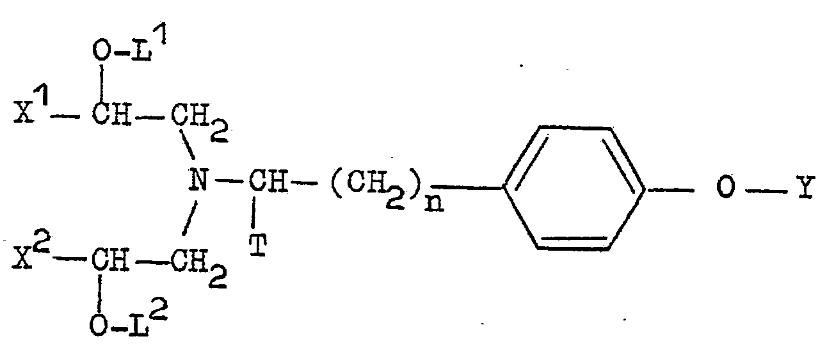

I

dans laquelle
n est égal à 1 ou 2,
$L^1$ et $L^2$ représentent l'hydrogène ou des groupes (alkyle ou alcoxy en C !–C#)-carbonyle,
T représente l'hydrogène ou un groupe méthyle,
$X^1$ et $X^2$ représentent des groupes phényle ou phényle monosubstitués en position m par Br, Cl, F, $CF_3$

ou $NO_2$,
Y représente un groupe –(CH2) ! _≏O–G, $-(CH_2)_{1-6}-CH=CH-C(O)-Z$, –C(O)–Z ou –CH(COOR")@,
G représente un groupe alkyle en C !–C$, (alcoxy en C !–C$)-alkyle en C !–C$ ou –(CH@) ! à $–Q,
Q représente un groupe phénoxy, phényle ou p-fluoro- ou p-phénoxyphényle,
Z représente un groupe OR ou N(R,R') et
R et R' représentent l'hydrogène ou des groupes alkyle en C !–C$ ou bien
R et R' forment ensemble et avec l'atome d'azote auquel ils sont reliés et le cas échéant avec un atome d'oxygène ou un atome d'azote supplémentaire, un cycle saturé à 5 ou 6 chaînons, et
R" représente un groupe alkyle en C !–C$, et de leurs sels acceptables pour l'usage pharmaceutique, caractérisé en ce que

a) on éthérifie un phénol correspondant à l'éther de phénol de formule I à l'aide d'un agent introduisant le groupe Y, ou bien

b) on alkyle une amine de formule

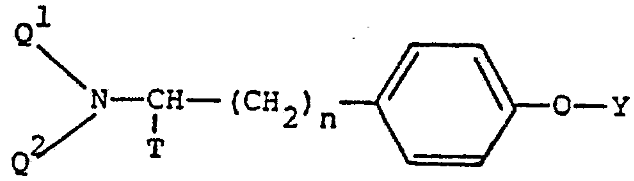

II

dans laquelle l'un des symboles $Q^1$ et $Q^2$ représente l'hydrogène et l'autre l'hydrogène ou un groupe Q de formule

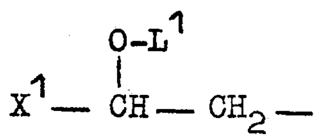

ou

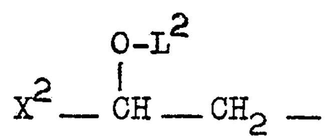

à l'aide d'un agent introduisant le groupe Q et

c) si on le désire, on soumet un substituant réactif contenu dans un groupe Y du produit de réaction à une modification fonctionnelle, si on le désire, on estérifie un groupe hydroxy ou les deux groupes hydroxy en position bêta de l'atome d'azote aminé, et si on le désire on convertit un composé de formule I en un sel.

2. Procédé selon les revendications 1a et 1c, dans lequel G représente un groupe alcoxyalkyle en C !–C$.

3. Procédé selon la revendication 1 ou 2, dans lequel n est égal à 1.

4. Procédé selon la revendication 1, 2 ou 3, dans lequel $L^1$ et $L^2$ représentent des groupes acétyle, éthoxycarbonyle ou plus spécialement l'hydrogène.

5. Procédé selon l'une des revendications 1 à 4, dans lequel T représente un groupe méthyle, et plus spécialement dans lequel l'atome de carbone sur lequel le groupe méthyle T est fixé est en configuration R.

6. Procédé selon l'une des revendications 1 à 5, dans lequel $X^1$ et $X^2$ représentent des groupes phényle, m-fluorophényle ou plus spécialement m-chlorophényle, et spécialement dans lequel l'atome de carbone sur lequel $X^1$ est fixé est en configuration R et l'atome de carbone sur lequel $X^2$ est fixé est en configuration R ou S.

7. Procédé selon l'une des revendications 1 à 6, dans lequel Y représente un groupe 2-méthoxyéthoxyéthyle, 2-p-fluorophénéthoxyéthyle, 2-phénoxyéthoxyéthyle, p-phénoxybenzyloxyéthyle, diméthylcarbamoyle, méthoxycarbonylallyle, bis-(méthoxycarbonyl)-méthyle, éthoxycarbonyle ou plus spécialement 2-phénéthoxyéthyle ou 2-éthoxyéthyle.

8. Procédé selon l'une des revendications 1 à 7, dans lequel n est égal à 1; $L^1$ et $L^2$ représentent l'hydrogène; T représente l'hydrogène ou un groupe méthyle; $X^1$ et $X^2$ représentent des groupes m-chlorophényle et Y représente un groupe 2-phénéthoxyéthyle ou 2-éthoxyéthyle, et plus spécialement dans lequel l'atome de carbone sur lequel $X^1$ est fixé est en configuration R et l'atome de carbone sur lequel $X^2$ est fixé est en configuration R ou S.

9. Procédé selon l'une des revendications 1 à 8 caractérisé en ce que l'on prépare l'alcool α,α'-[[[p-(2-éthoxyéthoxy)-phénéthyl]-imino]-diméthylène]-bis-[(R)-m-chlorobenzylique].

10. Procédé selon l'une des revendications 1 à 8, caractérisé en ce que l'on prépare un composé du groupe des suivants:
l'alcool α,α'-[[[(R)-α-méthyl-p-[2-(phénéthoxy)éthoxy]-phénéthyl]-imino]-diméthylène]-bis-[(R)-m-chlorobenzylique],

l'alcool (R)-m-chloro-α-[[[(S)-m-chloro-β-hydroxyphénéthyl]-(R)-α-méthyl-p-[2-(phénétoxy)-éthoxy]-phénéthyl]-amino]-méthyl]-benzylique,
l'alcool 3-chloro-α,α'-[[[(R)-p-(2-éthoxyéthoxy)-α-méthyl-phénéthyl]-imino]-diméthylène]-di-(R)-benzylique,
l'alcool (S)-m-chloro-α-[[[(R)-p-(2-éthoxyéthoxy)-α-méthyl-phénéthyl]-[(R)-β-hydroxyphénéthyl]-amino]-méthyl]-benzylique,
l'alcool (R)-m-chloro-α-[[[(S)-m-chloro-β-hydroxyphénéthyl]-[p-(2-éthoxyéthoxy)-phénéthyl]-amino]-méthyl]-benzylique et
l'alcool (R)-m-chloro-α-[[[(S)-m-chloro-β-hydroxyphénéthyl]-[p-(2-phénéthoxyéthoxy)-phénéthyl]-amino]-méthyl]-benzylique.